(19) **Europäisches Patentamt**

**European Patent Office**

**Office européen des brevets**

(11) **EP 1 140 779 B1**

(12) **EUROPÄISCHE PATENTSCHRIFT**

(45) Veröffentlichungstag und Bekanntmachung des Hinweises auf die Patenterteilung:
**17.03.2004 Patentblatt 2004/12**

(51) Int Cl.[7]: **C07C 68/00**, C07C 69/96

(21) Anmeldenummer: **99963454.6**

(22) Anmeldetag: **09.12.1999**

(86) Internationale Anmeldenummer:
**PCT/EP1999/009696**

(87) Internationale Veröffentlichungsnummer:
**WO 2000/037413 (29.06.2000 Gazette 2000/26)**

(54) **VERFAHREN ZUR HERSTELLUNG VON DIARYLCARBONATEN**

METHOD FOR PRODUCING DIARYL CARBONATES

PROCEDE DE PRODUCTION DE DIARYLCARBONATES

(84) Benannte Vertragsstaaten:
**AT BE CH CY DE DK ES FI FR GB GR IE IT LI LU MC NL PT SE**

(30) Priorität: **22.12.1998 DE 19859290**

(43) Veröffentlichungstag der Anmeldung:
**10.10.2001 Patentblatt 2001/41**

(73) Patentinhaber: **Bayer MaterialScience AG
51368 Leverkusen (DE)**

(72) Erfinder:
• **REISINGER, Claus-Peter**
**D-47798 Krefeld (DE)**
• **JANSEN, Ursula**
**D-47799 Krefeld (DE)**
• **RECHNER, Johann**
**D-47906 Kempen (DE)**
• **EEK, Rob**
**D-51061 Köln (DE)**

(56) Entgegenhaltungen:
**EP-A- 0 749 955        EP-A- 0 801 051**

EP 1 140 779 B1

**Beschreibung**

Es ist bekannt, organische Carbonate durch oxidative Umsetzung einer aromatischen Hydroxyverbindung mit Kohlenmonoxid in Gegenwart eines Edelmetall-Katalysators und herzustellen (DE-OS 27 38 437). Als Edelmetall wird bevorzugt Palladium eingesetzt. Zusätzlich können ein Cokatalysator (z.B. Mangan- oder Kobaltsalze), eine Base, ein quaternäres Salz, verschiedene Chinone bzw. Hydrochinone und Trockenmittel eingesetzt werden. Dabei kann in einem Lösungsmittel, bevorzugt Methylenchlorid, gearbeitet werden.

Bei der Umsetzung aromatischer Diydroxyverbindungen mit Kohlenmonoxid und Sauerstoff wird pro Mol gebildeter Carbonateinheit ein Mol Wasser frei. Verbleibt dieses Wasser im Reaktionssystem, kann bereits gebildetes organisches Carbonat hydrolysiert werden, so daß die erzielbaren Raum-Zeit-Ausbeuten ohne eine effektive Wasserabtrennung nur gering sind, außerdem kann das Katalysatorsystem durch Wasser desaktiviert werden. Die Reaktivierung des desaktivierten Katalysators erfordert aber einen hohen technischen Aufwand. Der Ersatz des desaktivierten Katalysators durch frischen ist mit hohen Kosten verbunden. Aus diesen Gründen ist eine effektive Wasserentfernung für die wirtschaftliche Nutzung dieses Prozesses essentiell.

In DE-OS 27 38 437 wird zur Wasserabtrennung der Zusatz von Molsieb vorgeschlagen. Die Verwendung von Molsieb macht eine technische Nutzung des Verfahrens jedoch unattraktiv, da für eine effektive Abtrennung des Wassers aus der Flüssigphase große Mengen Molsieb (100 - 500% Überschuß) benötigt werden, die unter hohem technischen Aufwand regeneriert werden müssen.

Aus US-A 5,498,724 geht ein Verfahren hervor, bei dem das bei der Reaktion gebildete Wasser durch Strippen mit überschüssigem Reaktionsgas entfernt wird. Das Reaktionsgas kann 0 bis 30 Vol.-% eines Inertgases enthalten, das mit Wasser ein Azeotrop bildet. In der Reaktionsmischung können auch inerte organische Lösungsmittel enthalten sein. Bei diesem Verfahren muß jedoch mit großen Gasmengen gearbeitet werden, um das Wasser vollständig zu entfernen; der erreichbare minimale Wassergehalt liegt oberhalb von 500 ppm. Bei der Wasserentfernung werden auch größere Mengen an aromatischer Hydroxyverbindung ausgetragen, die aus dem Gasstrom abgetrennt werden müssen.

EP-A 0 749 955 offenbart ein Verfahen zur Herstellung von Diarylcarbonaten, bei dem Wasser unter reduziertem Druck entfernt wird.

Es wurde nun überraschend gefunden, daß der Zusatz inerter organischer Lösungsmittel zum Reaktionsgemisch, die unter den Reaktionsbedingungen ein Azeotrop mit Wasser bilden, und die Entfernung dieses Azeotrops aus dem Reaktionsgemisch es ermöglicht, den Wassergehalt in der Reaktionsmischung auf deutlich weniger als 500 ppm, bevorzugt sogar unter 250 ppm, einzustellen. Zudem kann mit wesentlich kleineren Reaktionsgasmengen gearbeitet werden als beim reinen Strippen mit Reaktionsgas (DE-OS 44 03 075). Dadurch können bei einer großtechnischen Umsetzung des Verfahrens erhebliche Kosteneinsparungen erreicht werden.

Gegenstand der Erfindung ist somit ein Verfahren zur Herstellung eines aromatischen Carbonats der Formel

$$R\text{-}O\text{-}CO\text{-}O\text{-}R \qquad (I),$$

in der

R substituiertes oder nicht substituiertes $C_6$-$C_{12}$-Aryl, bevorzugt substituiertes oder nicht substituiertes Phenyl, besonders bevorzugt nicht substituiertes Phenyl bedeutet,

durch Umsetzung einer aromatischen Hydroxyverbindung der Formel

$$R\text{-}O\text{-}H \qquad (II),$$

worin R die oben angegebene Bedeutung hat, mit Kohlenmonoxid und Sauerstoff in Gegenwart eines Platinmetall-Katalysators, eines Cokatalysators, einer Base, und gegebenenfalls eines quaternären Salzes sowie eines inerten organischen Lösungsmittels, wobei das inerte organische Lösungsmittel unter den Reaktionsbedingungen ein Azeotrop mit dem bei der Reaktion entstehenden Wasser bildet, und dieses Azeotrop aus dem Reaktionsgemisch entfernt wird.

In einer bevorzugten Ausführungsform wird die Entfernung des Wassers aus dem Reaktionsgemisch als Azeotrop mit dem Lösemittel durch Strippen mit überschüssigen Reaktionsgas unterstützt. Dabei ist entscheidend, daß mehr als 5 Vol.-% des Lösemittels in Form des Azeotrops aus der Reaktionsmischung entfernt werden. In einer bevorzugten Ausführungsform wird die Entfernung des Azeotrops aus dem Reaktionsgemisch durch überschüssiges Reaktionsgas unterstützt. Unter reinen Strippbedingungen (DE-OS 44 03 075), d.h. ohne Azeotropbildung mit dem inerten, organischen Lösemittel und gleichzeitiger Entfernung des Azeotrops aus dem Reaktionsgemisch, werden deutlich geringere Umsätze erzielt.

Das erfindungsgemäße Verfahren zur Carbonatbildung wird bei einer Reaktionstemperatur von 30 bis 200°C, bevorzugt 50 bis 150°C, besonders bevorzugt 60 bis 130°C, und bei einem Reaktionsdruck von 1 bis 100 bar, bevorzugt 1 bis 50 bar, besonders bevorzugt 1 bis 10 bar durchgeführt. Die Temperatur und der Gesamtdruck sind so zu wählen, daß das Azeotrop unter den Reaktionsbedingungen gebildet und teilweise aus dem Reaktionsgemisch entfernt werden kann.

Als inertes organisches Lösungsmittel können geeignet siedende, mit Wasser azeotropbildende halogenierte Kohlenwasserstoffe und aromatische Lösemittel wie Chlorbenzol, Dichlorbenzol, Fluorbenzol, Benzol, Anisol, Methylenchlorid oder 1,2-Dichlorethan, ggf. auch Mischungen davon, verwendet werden. Besonders bevorzugt wird Chlorbenzol eingesetzt. Das inerte Lösemittel kann mit einem Anteil von 1-99 %, bevorzugt 20-98 %, besonders bevorzugt 40-98 %, in der Reaktionsmischung enthalten sein.

Durch ein im azeotrophaltigen Abgasstrom befindliches Trennorgan, wie z.B. einen Dephlegmator, eine Destillationskolonne mit Böden oder Packung und weitere dem Fachmann bekannte Apparate, kann der größte Teil des bei der Entwässerung mitgerissenen Lösungsmittels vom Wasser abgetrennt und in den Rückstrom zum Reaktor geführt werden. Die Trennung bzw. Brechung des abgetrennten Azeotrops kann nach dem Stand der Technik z.B. durch Extraktion, Ausfrieren oder Destillation erfolgen.

Der mit dem Azeotrop ausgetriebene Anteil an gelösten Gasen läßt sich in einer bevorzugten Ausführungsform nach der Abtrennung wieder dem Reaktorkreisgas zuführen. Die Abtrennung mitgerissener Edukte (z.B. Phenol), Lösungsmittel, Produkte und Wasser aus dem zu recyclisierenden ggf. vor der Trennung komprimierten Gasgemisch erfolgt nach dem Stand der Technik, z.B. durch Adsorption, Absorption oder bevorzugt durch Kondensation. Das zur Reaktion benötigte Reaktionsgas, bestehend aus Kohlenmonoxid, Sauerstoff und einem Inertgas, wird hierzu in einer Menge von 1 bis 10000 N1 pro Liter Reaktionslösung, bevorzugt 5 bis 5000 N1 pro Liter Reaktionslösung und besonders bevorzugt 10 bis 1000 N1 pro Liter Reaktionslösung eingeleitet. Das aus der Entwässerung stammende, zu recyclisierende Gasgemisch wird bezüglich seiner Anteile an CO und $O_2$ auf die genannten Volumina angerechnet.

Die Zusammensetzung der Reaktionsgase Kohlenmonoxid und Sauerstoff kann in weiten Konzentrationsgrenzen variiert werden, es wird jedoch zweckmäßigerweise ein $CO:O_2$-Molverhältnis (normiert auf CO) von 1:(0,001-1,0) bevorzugt 1:(0,01- 0,5) und besonders bevorzugt von 1:(0,02-0,3) eingestellt. Der Sauerstoffpartialdruck ist bei diesen Molverhältnissen groß genug, um hohe Raum-Zeit-Ausbeuten erreichen zu können und gleichzeitig werden explosionsfähige Kohlenmonoxid/Sauerstoff-Gasgemische vermieden.

Alle Ausgangsverbindungen können mit Verunreinigungen aus ihrer Herstellung und Lagerung-kontaminiert sein, jedoch ist es im Sinne der Reinheit des Endproduktes wünschenswert, mit möglichst sauberen Chemikalien zu arbeiten. Auch die Reaktionsgase unterliegen keinen besonderen Reinheitsanforderungen. So kann Synthesegas als CO-Quelle und Luft als $O_2$-Träger dienen, es ist jedoch darauf zu achten, daß keine Katalysatorgifte wie z.B. Schwefel oder dessen Verbindungen eingetragen werden. In der bevorzugten Ausführungsform des erfindungsgemäßen Verfahrens werden reines CO und reiner Sauerstoff verwendet.

Bei den erfindungsgemäß umsetzbaren aromatischen Hydroxyverbindungen handelt es sich beispielsweise um Phenol, o-, m- oder p-Kresol, o-, m- oder p-Chlorphenol, o-, m- oder p-Ethylphenol, o-, m- oder p-Propylphenol, o-, m- oder p-Methoxyphenol, 2,6-Dimethylphenol, 2,4-Dimethylphenol, 3,4-Dimethylphenol, 1-Naphthol, 2-Naphthol und Bisphenol A, bevorzugt um Phenol. Allgemein handelt es sich im Falle einer Substitution der aromatischen Hydroxyverbindung um 1 oder 2 Substituenten in der Bedeutung von $C_1$-$C_4$-Alkyl, $C_1$-$C_4$-Alkoxy, Fluor, Chlor oder Brom.

In das erfindungsgemäße Verfahren einsetzbare Basen stellen Alkalihydroxide, Alkalisalze bzw, quaternäre Salze von schwachen Säuren wie Alkalitert.-butylate oder Alkalisalze bzw. quaternäre Salze von aromatischen Hydroxyverbindungen der Formel (II) dar, in der R die oben angegebene Bedeutung hat. Ganz besonders bevorzugt wird ein Alkalisalz bzw. quaternäre Salz der aromatischen Hydroxyverbindung der Formel (II) verwendet, die auch zum organischen Carbonat umgesetzt werden soll, beispielsweise Tetrabutylammoniumphenolat. Diese Alkalisalze können Lithium-, Natrium-, Kalium-, Rubidium- oder Cäsiumsalze sein. Bevorzugt werden Lithium-, Natrium-, und Kaliumphenolate, besonders bevorzugt Kaliumphenolat eingesetzt.

Die Base wird in katalytischen Mengen zugesetzt. Das Verhältnis von Platinmetall, z.B. Palladium, zu Base wird vorzugsweise so gewählt, daß pro Grammatom Platinmetall, z.B. Palladium, 0,1 bis 500, bevorzugt 0,3 bis 200 besonders bevorzugt 0,9 bis 130 Äquivalente Base eingesetzt werden.

Die für das erfindungsgemäße Verfahren geeigneten Platinmetall-Katalysatoren bestehen aus mindestens einem Edelmetall der Gruppe VIII, vorzugsweise Palladium. Es kann bei dem erfindungsgemäßen Verfahren in verschiedener Form zugegeben werden.

Palladium kann in metallischer Form oder bevorzugt in Form von Palladium-Verbindungen der Oxidationsstufen 0 und +2, wie beispielsweise Palladium (II)-acetylacetonat, -halogenide, -carboxylate von $C_2$-$C_{18}$-Carbonsäuren, -nitrat, -oxide oder Palladiumkomplexe, die beispielsweise Olefine, Amine, Phosphorverbindungen und Halogenide enthalten können, eingesetzt werden. Besonders bevorzugt sind Palladiumbromid und Palladiumacetylacetonat.

Die Menge an Platinmetall-Katalysator ist im erfindungsgemäßen Verfahren nicht beschränkt. Bevorzugt wird so viel Katalysator zugesetzt, daß die Konzentration des Metalls im Reaktionsansatz 1 - 3000 ppm beträgt, besonders bevorzugt sind Konzentrationen von 5 - 500 ppm.

Als Cokatalysator für das erfindungsgemäße Verfahren wird ein Metall der Gruppen III A, III B, IV A, IV B, V B, I B, II B, VI B, VII B, der Seltenerdmetalle (Atomnummern 58-71) oder der Eisengruppe des Periodensystems der Elemente (Mendelejew), ggf. auch Mischungen davon, verwendet, wobei das Metall in verschiedenen Oxidationsstufen eingesetzt werden kann. Bevorzugt werden Mn, Cu, Co, V, Zn, Ce und Mo eingesetzt. Ohne das erfindungsgemäße Verfahren einzuschränken, seien Mangan(II), Mangan(III), Kupfer(I), Kupfer(II), Kobalt(II), Kobalt(III), Vanadium(III) und Vanadium(IV) genannt. Die Metalle können beispielsweise als Halogenide, Oxide, Carboxylate von $C_2$-$C_6$-Carbonsäuren, Diketonate oder Nitrate sowie als Komplexverbindungen eingesetzt werden, die beispielsweise Kohlenmonoxid, Olefine, Amine, Phosphorverbindungen und Halogenide enthalten können. Besonders bevorzugt werden Mn, Cu, Mo und Ce eingesetzt. Ganz besonders bevorzugt werden Manganverbindungen im erfindungsgemäßen Verfahren verwendet, besonders bevorzugt Mangan(II)- und Mangan(III)komplexe, ganz besonders bevorzugt Manoan(II)acetylacetonat bzw. Mangan(III)-acetylacetonat.

Der Cokatalysator, der auch in-situ gebildet werden-kann, wird in einer solchen Menge zugesetzt, daß seine Konzentration im Bereich von 0,0001 bis 20 Gew.-% des Reaktionsgemisches liegt, bevorzugt ist der Konzentrationsbereich von 0,005 bis 5 Gew.-%, besonders bevorzugt 0,01 bis 2 Gew.-%.

Bei den im Rahmen der vorliegenden Erfindung eingesetzten quaternären Salzen kann es sich beispielsweise um mit organischen Resten substituierte Ammonium-, Guanidinium-, Phosphonium- oder Sulfoniumsalze, ggf. auch Mischungen davon, handeln. Geeignet für den Einsatz in das erfindungsgemäße Verfahren sind Ammonium-, Guanidinium-, Phosphonium- und Sulfoniumsalze, die als organische Reste $C_6$- bis $C_{10}$-Aryl-, $C_7$- bis $C_{12}$-Aralkyl- und/oder $C_1$- bis $C_{20}$-Alkyl-Reste und als Anion ein Halogenid, Tetrafluoroborat oder Hexafluorophosphat tragen. Bevorzugt werden in das erfindungsgemäße Verfahren Ammoniumsalze eingesetzt, die als organische Reste $C_6$- bis $C_{10}$-Aryl-, $C_7$- bis $C_{12}$-Aralkyl- und/oder $C_1$- bis $C_{20}$-Alkyl-Reste und als Anion ein Halogenid tragen, besonders bevorzugt sind Tetrabutylammoniumbromid und Tetrabutylphosphoniumbromid. Die Menge eines solchen quatemären Salzes kann beispielsweise 0,1 - 20 Gew.-%, bezogen auf das Gewicht des Reaktionsgemisches, betragen. Vorzugsweise beträgt diese Menge 0,5 - 15 Gew.-%, besonders bevorzugt 1 - 5 Gew.-%.

Bei Verwendung einer Base wie Tetrabutylammoniumphenolat, welche ein quatemäres Kation enthält, kann die Menge an zugesetztem quaternären Salz wie Tetrabutylammoniumbromid entsprechend verringert werden. Gegebenenfalls kann man die Gesamtmenge des Anions des zugesetzten quaternären Salzes auch durch andere Salze dieses Anions wie Kaliumbromid ausgleichen.

In einer weiteren Ausführungsform werden statt des homogenen Katalysatorsystems heterogene Katalysatoren, bei denen das Platinmetall oder das Platinmetall und der Cokatalysator auf einem heterogenen Träger aufgebracht sind, als Pulver oder Formkörper eingesetzt. Die übrigen Komponenten des Katalysatorsystems, wie die Base, die quaternäre Verbindung und gegebenenfalls der Cokatalysator, sind weiterhin in der Reaktionslösung homogen gelöst. Die Menge des Platinmetalls am Gesamtgewicht des heterogenen Katalysators beträgt 0,01 bis 15 Gew.-%, bevorzugt 0,05 bis 10 Gew.-%, gerechnet als Platinmetall.

Als Cokatalysatoren auf dem Katalysatorträger wird mindestens eine Metallverbindung der oben genannten Art eingesetzt.

Die Menge des Cokatalysators am Gesamtgewicht des heterogenen Katalysators beträgt 0,01 bis 15 Gew.-%, bevorzugt 0,05 bis 10 Gew.-% gerechnet als Metall.

Als Katalysatorträger eignen sich ein oder mehrere Metalloxide aus der Gruppe von V, Mn, Ti, Cu, Zr, La, der Seltenerdmetalle (Atomnummern 58-71), sowohl im Sinne chemisch einheitlicher Reinsubstanzen als auch im Gemisch sowie Eisen- und Kobaltoxide, Nikkel-, Aluminium-, Silizium- und Magnesiumoxid, Zeolithe und Aktivkohlen. Wird der Trägerkatalysator als Pulver eingesetzt, sind zur Vermischung der Reaktionskomponenten die zu verwendenden Rührbehälter mit dafür brauchbaren Rührern ausgestattet, bzw. als Blasensäulenreaktor gestaltet.

Beim Arbeiten mit Trägerkatalysator-Pulvern als Suspension in Rührgefäßen, oder Blasensäulen werden Mengen von 0,001 bis 50 Gew.-%, bevorzugt von 0,01 bis 20 Gew.-%, besonders bevorzugt von 0,1 bis 10 Gew.-% Trägerkatalysator-Pulver auf die eingesetzte Menge an aromatischer Hydroxyverbindung, verwendet.

In bevorzugten Ausführungsformen wird der heterogene Trägerkatalysator ortsfest in Rührbehältern, einer Blasensäule, einem Rieselphasenreaktor oder Kaskaden dieser Reaktoren eingesetzt. Eine Abtrennung des Trägerkatalysators entfällt dann völlig.

Als Reaktoren für das erfindungsgemäße Verfahren mit homogenem oder heterogenem Katalysator sind Rührkessel, Autoklaven und Blasensäulen geeignet, wobei diese als Einzelreaktoren oder als Kaskade eingesetzt werden können. In einer Kaskade können 2 bis 15, bevorzugt 2 bis 10, besonders bevorzugt 2 bis 5 Reaktoren hintereinandergeschaltet sein.

Zur Vermischung der Reaktionskomponenten sind die erfindungsgemäß zu verwenden Rührbehälter mit dafür geeigneten Rührer ausgestattet. Solche Rührer sind dem Fachmann bekannt. Es seien beispielhaft genannt: Scheiben-, Impeller-, Propeller-, Schaufel-, MIG- und Intermig-Rührer, Rohrrührer und verschiedene Hohltührertypen. Bevorzugte Rührer sind solche, die eine effektive Vermischung von Gasen und Flüssigkeiten erlauben, beispielsweise Hohlrohrbegasungsrührer, Propellerrührer etc.

Als Blasensäulen können in dem erfindungsgemäßen Verfahren folgende Typen, eingesetzt werden: einfache Blasensäulen, Blasensäulen mit Einbauten, wie z.B.: Blasensäulen mit parallelen Kammern, Kaskaden-Blasensäulen mit Siebböden oder Einlochböden, Blasensäulen mit Packungen, mit statischen Mischern, pulsierende Siebbodenblasensäulen, Schlaufenreaktoren wie z.B.: Mammutschlaufenreaktoren, Abstromschlaufenreaktoren, Strahlschlaufenreaktor, Freistrahlreaktoren, Strahldüsenreaktoren, Blasensäulen mit Flüssigkeitstauchstrahler, Abstrom-Aufstrom-Blasensäulen und weitere dem Fachmann bekannte Blasensäulenreaktoren (Chem. Ing. Tech. 51 (1979) Nr. 3, S. 208-216; W.-D. Deckwer, Reaktionstechnik in Blasensäulen, Otto Salle Verlag 1985).

In einer bevorzugten Ausführungsform kommen Blasensäulenreaktoren und Blasensäulen-Kaskaden zum Einsatz, die eine effektive Vermischung von Gas und Flüssigkeiten erlauben, wie beispielsweise Kaskaden-Blasensäulen und Schlaufenreaktoren. Zur Aufrechterhaltung einer guten Durchmischung von Flüssigkeit und Reaktionsgas können Verteilungs- und Redispergierorgane entlang der Längsachse der Blasensäulenreaktoren angebracht sein. Als feste Redispergierorgane kommen Einlochböden, Lochplatten, Siebböden, sowie weitere dem Fachmann bekannte Einbauten zum Einsatz. Für die Erstdispergierung des Reaktionsgases in der flüssigen Phase bei der Eindosierung sind übliche Vorrichtungen wie poröse Sinterplatten, Lochplatten, Siebböden, Einsteckrohre, Düsen, Begasungsringe und weitere dem Fachmann bekannte Dispergiervorrichtungen einsetzbar.

Das erfindungsgemäße Verfahren kann in verschiedenen Ausführungsvarianten ausgeübt werden. Eine Möglichkeit besteht in der diskontinuierlichen Durchführung. Dabei werden CO und Sauerstoff entweder durch einen Begasungsrührer wie im Falle eines Rührkessels oder durch andere bekannte Gasverteilungsorgane in die Reaktionsmischung geleitet. Nach Erreichen des optimalen Umsatzes wird das Reaktionsgemisch aus dem Reaktor entfernt oder gegebenenfalls im Reaktor aufgearbeitet. Im Falle der Verwendung pulverförmigen Trägerkatalysatoren können diese aus der Reaktionsmischung z.B. durch Filtration, Sedimentation oder Zentrifugieren abgetrennt werden.

In diskontinuierlichen Versuchen verwendete Trägerkatalysatoren können bei gleichen Einsatzstoffen ggf. ohne Reinigung wiederholt eingesetzt werden. Bei kontinuierlicher Arbeitsweise können die eingesetzten Trägerkatalysatoren über lange Zeit im Reaktor verbleiben und gegebenenfalls regeneriert werden.

In bevorzugter Weise kommt eine kontinuierliche Arbeitsweise im Einzelreaktor oder in einer Kaskade mehrerer Reaktoren zum Einsatz. Bei Verwendung ortsfester heterogener Katalysatoren können diese über lange Zeit im Reaktor verbleiben und dort auch ggf. regeneriert werden.

## Beispiele

### Beispiel 1

In einem 250 ml Autoklaven mit Begasungsrührer, Kühler und nachgeschalteter Kühlfalle wurden 0,3 mmol Palladiumbromid, 22 mmol Tetrabutylammoniumbromid und 20 g Phenol in 90 ml Chlorbenzol vorgelegt und 30 min bei 90 °C unter Einleitung von Kohlenmonoxid (3 l/h) gelöst. Anschließend wurden 2,2 mmol Mangan(III)acetylacetonat und 10,4 mmol Tetrabutylammoniumphenolat mit 10 ml Chlorbenzol zugeben, und unter Einleitung von 80 Nl/h eines Gasgemisches aus Kohlenmonoxid und Sauerstoff (95:5 Vol%) bei 3 bar Gesamtdruck und 110 °C die Reaktion gestartet. Dem Reaktionsgemisch wurde jede Stunde eine Probe entnommen und gaschromatographisch analysiert. Die Analysen ergaben, daß nach 1 h bereits 11,4 % Diphenylcarbonat und nach 2 h insgesamt 14,6 % Diphenylcarbonat im Reaktionsgemisch enthalten waren. In der Kühlfalle waren 12 g einer Clorbenzol/Wasser-Suspension kondensiert. Nach Versuchsende hatte die Reaktionsmischung einen Wasserrestgehalt von weniger als 250 ppm.

### Beispiel 2

In einem 250 ml Autoklaven mit Begasungsrührer, Kühler und nachgeschalteter Kühlfalle wurden 0,15 mmol Palladiumbromid, 11 mmol Tetrabutylammoniumbromid und 10 g Phenol in 90 ml Chlorbenzol vorgelegt und 30 min bei 90 °C unter Einleitung von Kohlenmonoxid (3 l/h) gelöst. Anschließend wurden 1,1 mmol Mangan(III)acetylacetonat und 5,2 mmol Kaliumphenolat mit 10 ml Chlorbenzol zugeben, und unter Einleitung von 80 Nl/h eines Gasgemisches aus Kohlenmonoxid und Sauerstoff (95:5 Vol.-%) bei 5 bar Gesamtdruck und 125 °C die Reaktion gestartet. Dem Reaktionsgemisch wurde jede halbe Stunde eine Probe entnommen und gaschromatographisch analysiert. Die Analysen ergaben, daß nach 0.5 h 5,4 % Diphenylcarbonat und nach 1 h insgesamt 7,2 % Diphenylcarbonat im Reaktionsgemisch enthalten waren. In der Kühlfalle waren 10 g einer Clorbenzol/Wasser-Suspension kondensiert. Nach Versuchsende hatte die Reaktionsmischung einen Wasserrestgehalt von weniger als 250 ppm.

### Beispiel 3 (Vergleich)

In einem 250 ml Autoklaven mit Begasungsrührer, Kühler und nachgeschalteter Kühlfalle wurden 0,15 mmol Palladiumbromid, 11 mmol Tetrabutylammoniumbromid und 10 g Phenol in 90 ml Chlorbenzol vorgelegt und 30 min bei 90 °C unter Einleitung von Kohlenmonoxid (3 l/h) gelöst. Anschließend wurden 1,1 mmol Mangan(III)acetylacetonat und 5,2 mmol Kaliumphenolat mit 10 ml Chlorbenzol zugeben, und unter Einleitung von 80 Nl/h eines Gasgemisches aus Kohlenmonoxid und Sauerstoff (95:5 Vol.-%) bei 10 bar Gesamtdruck und 125 °C die Reaktion gestartet. Dem Reaktionsgemisch wurde jede halbe Stunde eine Probe entnommen und gaschromatographisch analysiert. Die Analysen ergaben, daß nach 0.5 h 0,9 % Diphenylcarbonat und nach 1 h insgesamt 1,2 % Diphenylcarbonat im Reaktionsgemisch enthalten waren. In der Kühlfalle waren weniger als 2 g Wasser kondensiert. Nach Versuchsende hatte die Reaktionsmischung einen Wasserrestgehalt von über 500 ppm. Die verwendete Einstellung von Druck und Temperatur entsprechen reinen Strippbedingungen, da das Azeotrop praktisch nicht aus dem Reaktionsgemisch entfernt wurde. Der direkte Vergleich mit Beispiel 2 belegt die Effizienz der Entfernung des Azeotrops aus dem Reaktionsgemisch.

**Patentansprüche**

1.  Verfahren zur Herstellung eines aromatischen Carbonats der Formel

$$R\text{-}O\text{-}CO\text{-}O\text{-}R \qquad (I),$$

in der

R   substituiertes oder nicht substituiertes $C_6$-$C_{12}$-Aryl, bevorzugt substituiertes oder nicht substituiertes Phenyl, besonders bevorzugt nicht substituiertes Phenyl bedeutet,

bei dem eine aromatische Hydroxyverbindung der Formel

$$R\text{-}O\text{-}H \qquad (II),$$

worin R die oben angegebene Bedeutung hat, mit Kohlenmonoxid und Sauerstoff in Gegenwart eines Platinmetall-Katalysators, eines Cokatalysators, einer Base, **dadurch gekennzeichnet, daß** ein inertes organisches Lösungsmittel verwendet wird, welches unter den Reaktionsbedingungen ein Azeotrop mit dem bei der Reaktion entstehenden Wasser bildet, und dieses Azeotrop aus dem Reaktionsgemisch entfernt wird.

2.  Verfahren gemäß Anspruch 1, bei dem die Entfernung des Azeotrops aus dem Reaktionsgemisch durch überschüssiges Reaktionsgas unterstützt wird.

3.  Verfahren gemäß Anspruch 1 oder 2, bei dem als inertes organisches Lösungsmittel aromatische Lösemittel eingesetzt werden.

4.  Verfahren gemäß Anspruch 3, bei dem als inertes organisches Lösungsmittel Chlorbenzol eingesetzt wird.

5.  Verfahren gemäß Anspruch 1 oder 2, bei dem als inertes organisches Lösungsmittel halogenierte Kohlenwasserstoffe eingesetzt werden.

6.  Verfahren nach Anspruch 5, bei dem als Lösungsmittel 1,2-Dichlorethan verwendet wird.

7.  Verfahren nach einem der Ansprüche 1 bis 6, bei dem als Basen Alkalisalze oder quatemäre Salze schwacher Säuren verwendet werden.

8.  Verfahren nach Anspruch 7, bei dem als Base Kaliumphenolat verwendet wird.

9.  Verfahren nach Anspruch 7, bei dem als Base Kalium-tert.-butylat verwendet wird.

10. Verfahren nach einem der Ansprüche 1 bis 6, bei dem als Basen Alkalihydroxide verwendet werden.

**Claims**

1.  Process for the production of an aromatic carbonate of the formula

$$R\text{-}O\text{-}CO\text{-}O\text{-}R \qquad (I),$$

in which

R   means a substituted or unsubstituted $C_6$-$C_{12}$ aryl, preferably substituted or unsubstituted phenyl, particularly preferably unsubstituted phenyl,

in which an aromatic hydroxy compound of the formula

$$R\text{-}O\text{-}H \qquad (II),$$

in which R has the above-stated meaning, is reacted with carbon monoxide and oxygen in the presence of a platinum group metal catalyst, a co-catalyst, a base, **characterized in that** an inert organic solvent is used, which, under the reaction conditions, forms an azeotrope with the water arising during the reaction, and this azeotrope is removed from the reaction mixture.

2.  Process according to claim 1, in which removal of the azeotrope from the reaction mixture is promoted by excess reaction gas.

3. Process according to claim 1 or 2, in which aromatic solvents are used as the inert organic solvent.

4. Process according to claim 3, in which chlorobenzene is used as the inert organic solvent.

5. Process according to claim 1 or 2, in which halogenated hydrocarbons are used as the inert organic solvent.

6. Process according to claim 5, in which 1,2-dichloroethane is used as the solvent.

7. Process according to any one of claims 1 to 6, in which alkali metal salts or quaternary salts of weak acids are used as the bases.

8. Process according to claim 7, in which potassium phenolate is used as the base.

9. Process according to claim 7, in which potassium tert.-butylate is used as the base.

10. Process according to any one of claims 1 to 6, in which alkali metal hydroxides are used as the bases.

**Revendications**

1. Procédé de préparation d'un carbonate aromatique de la formule :

$$R - O - CO - O - R \qquad (I)$$

dans laquelle
R représente un radical aryle en $C_6$-$C_{12}$ substitué ou non substitué, de préférence le radical phényle substitué ou non substitué, de manière particulièrement préférée, le radical phényle non substitué,

dans lequel on fait réagir un composé hydroxylé de la formule :

$$R - O - H \qquad (II)$$

où R a la signification donnée ci-dessus, avec du monoxyde de carbone et de l'oxygène en présence d'un catalyseur de métal du type platine, d'un cocatalyseur, d'une base, **caractérisé en ce que** l'on utilise un solvant organique inerte, qui forme un azéotrope avec l'eau formée dans la réaction, dans les conditions de réaction, et cet azéotrope est éliminé du mélange de réaction.

2. Procédé suivant la revendication 1, dans lequel l'élimination de l'azéotrope depuis le mélange réactionnel est facilitée par du gaz réactionnel en excès.

3. Procédé suivant la revendication 1 ou 2, dans lequel on met en oeuvre comme solvant organique, un solvant aromatique.

4. Procédé suivant la revendication 3, dans lequel on met en oeuvre comme solvant organique inerte, le chlorobenzène.

5. Procédé suivant la revendication 1 ou 2, dans lequel on met en oeuvre comme solvant organique inerte, un hydrocarbure halogéné.

6. Procédé suivant la revendication 5, dans lequel on met en oeuvre comme solvant, le 1,2-dichloroéthane.

7. Procédé suivant l'une quelconque des revendications 1 à 6, dans lequel on utilise comme base, un sel alcalin ou un sel quaternaire d'acide faible.

8. Procédé suivant la revendication 7, dans lequel on utilise comme base, le phénolate de potassium.

9. Procédé suivant la revendication 7, dans lequel on utilise comme base, le t-butylate de potassium.

10. Procédé suivant l'une quelconque des revendications 1 à 6, dans lequel on utilise comme base, un hydroxyde alcalin.